# FASCICULE DE BREVET EUROPEEN

(11) **EP 1 061 945 B1**
(45) Date de publication et mention de la délivrance du brevet: **05.06.2002**
(21) Numéro de dépôt: 99907701.9
(22) Date de dépôt: 12.03.1999
(51) Int. Cl.: A61K 38/22, A61P 25/00

(54) **UTILISATION D'ANALOGUES DU VIP DANS LA PREVENTION ET LE TRAITEMENT DES LESIONS CEREBRALES DU NOUVEAU-NE HUMAIN**
VERWENDUNG VON VIP ANALOGE FÜR VORBEUGUNG UND BEHANDLUNG VON GEHIRNBESCHÄDIGUNGEN VON MENSCHLICHEN NEUGEBORENEN
USE OF VIP ANALOGUES FOR PREVENTING AND TREATING BRAIN DAMAGE IN HUMAN BABIES

(30) Priorité: 13.03.1998 FR 9803125
(43) Date de publication de la demande: 27.12.2000
(73) Titulaire: ASSISTANCE PUBLIQUE - HOPITAUX DE PARIS, 75004 Paris RP (FR)
(72) Inventeur: GRESSENS, Pierre, F-75019 Paris (FR); ROBBERECHT, Patrick, B-1170 Bruxelles (BE)
(74) Mandataire: Vialle-Presles, Marie José
(86) Numéro de dépôt international: FR9900563
(87) Numéro de publication internationale: WO9947159

(56) Documents cités:
- EP-A- 0 620 008
- WO-A-97/29126
- GRESSENS P ET AL: "Vasoactive intestinal peptide prevents excitotoxic cell death in the murine developing brain." JOURNAL OF CLINICAL INVESTIGATION, (1997 JUL 15) 100 (2) 390-7, XP002086874 cité dans la demande
- WU J.Y. ET AL.: "Neurobehavioral development of neonatal mice following blockade of VIP during the early embryonic period" PEPTIDES, vol. 18, no. 8, 1997, pages 1131-1137, XP002086875
- HILL, JOANNA M. ET AL: "Blockade of VIP during neonatal development induces neuronal damage and increases VIP and VIP receptors in brain" ANN. N. Y. ACAD. SCI. (1994), 739(MODELS OF NEUROPEPTIDE ACTION), 211-225, XP002086876
- GRESSENS P: "[Vasoactive intestinal peptide: a novel neurotrophic factor]. Le peptide vasoactif intestinal: un nouveau facteur neurotrophique." ARCHIVES DE PEDIATRIE, (1998 JUN) 5 (6) 654-60, XP002112638
- ZUPAN V ET AL: "Involvement of pituitary adenylate cyclase-activating polypeptide II vasoactive intestinal peptide 2 receptor in mouse neocortical astrocytogenesis." JOURNAL OF NEUROCHEMISTRY, (1998 MAY) 70 (5) 2165-73. , XP002112639

## Description

La présente Invention est relative à une nouvelle utilisation d'analogues du VIP, dans la prévention et/ou le traitement des lésions cérébrales du nouveau-né humain.

On entend par « nouveau-né » au sens de la présente invention, aussi bien le foetus, le nouveau-né prématuré ou à terme que le jeune nourrisson.

Initialement, le peptide intestinal vasoactif (VIP) a été isolé et purifié, à partir de l'intestin de porc.

Ce peptide comprend 28 aminoacides :
HSDAVFTDNYTRLRKQMAVKKYLNSILN-NH₂(SEQ NO:1) et présente une homologie importante avec la sécrétine et le glucagon.

Des neurones contenant du VIP ont été localisés par immuno-test dans différents tissus : système endocrine, système exocrine, tissus musculaires lisses.

Le VIP présente de nombreuses activités biologiques, notamment au niveau du tractus gastro-intestinal et du système circulatoire; il présente en particulier une action de relaxation des muscles lisses. Il a été préconisé dans la relaxation des muscles lisses bronchiques et par voie de conséquence dans le traitement de l'asthme.

D'autres activités biologiques ont été décrites pour le VIP. En particulier, il a été montré (*Pierre Gressens et al*., *J*. *Clin. Investig*., *1997*, *100*, 2, *390-97*) que le VIP présente un rôle protecteur contre les lésions excitotoxiques induites par l'iboténate dans le cerveau de souris en développement

En effet, ces Auteurs ont montré que les lésions induites chez les rongeurs par l'injection d'iboténate miment certains désordres architecturaux néopalliaux observés en pathologie humaine, à divers stades de la grossesse.

Les lésions du cerveau humain apparaissent chez le nouveau-né à terme ou prématuré, généralement par manque d'oxygène ; ceci peut également survenir pendant la grossesse. Ce manque d'oxygène est une cause majeure de séquelles neurologiques.

Les différentes lésions qui ont pu être observées, sont essentiellement des lésions du cortex cérébral ou des lésions de la substance blanche (prématuré).

Le modèle chez la souris obtenu par injection intracérébrale d'iboténate reproduit :
- des lésions du cortex cérébral, semblables à celles observées chez le foetus humain entre 16 et 25 semaines de grossesse, lorsque l'iboténate est injecté le jour de la naissance (J_{N}),
- des lésions du cortex cérébral semblables aux lésions du nouveau-né humain à terme, lorsque l'injection d'iboténate est effectuée à J_{N} + 5 et
- des lésions de la substance blanche, semblables aux lésions du prématuré humain, lorsque l'iboténate est injecté à J_{N} + 5.

De manière plus précise :
. la dépopulation neuronale spécifique dans les couches V et Via rappelle la microgyrie observée entre 22 et 28 semaines après la conception chez la femme ;
. les lésions corticales diffuses et les kystes de la substance blanche constituent un modèle de lésions observées en période périnatale chez l'être humain: leucomalacie périventriculaire du prématuré et accident cortico-souscortical du nouveau-né à terme.

Le modèle décrit ci-dessus, qui constitue un bon modèle des lésions cérébrales du nouveau-né à terme ou prématuré humain, permet de tester des substances qui pourraient présenter un effet de neuro-protection dans le cadre des lésions cérébrales d'origine pré ou périnatales du nouveau-né prématuré et à terme.

*P. Gressens et al.* ont montré, dans l'article précité, qu'à J_{N} le VIP protège les lésions corticales et qu'à J_{N}+5, le VIP protège la substance blanche mais pas les lésions corticales. En effet, il semble que le VIP, injecté par voie intracérébrale, présente essentiellement une action préventive sur la polymicrogyrie et la leucomalacie périventriculaire.

Toutefois le VIP a l'inconvénient de n'être actif que par voie intracérébrale et seulement à titre préventif; cela présente donc un intérêt limité, pour une utilisation chez le prématuré ou le nouveau-né.

Les Inventeurs se sont en conséquence donné pour but de pourvoir à des composés qui puissent être administrés par voie générale (parentérale ou orale) et qui puissent avoir aussi bien une action préventive. qu'une action curative.

La présente Invention a pour objet l'utilisation d'un analogue du VIP pour l'obtention d'un médicament pour la prévention ou le traitement des lésions cérébrales du foetus, du nouveau-né prématuré ou à terme ou du jeune nourrisson humains, ledit analogue du VIP étant sélectionné dans le groupe constitué par les analogues cycliques du VIP et des dérivés du VIP non cycliques de 31 aminoacides comprenant en position N-terminale un groupe N-acétyle, puis Glu en position 8 (Glu⁸), Lys en position 12 (Lys¹²), norleucine en position 17 (Nle¹⁷), Ala en position 19 (Ala¹⁹), X1 en position 21, X2 en position 25, Leu en position 26 (Leu²⁶), Lys en positions 27 et 28 (Lys²⁷ et Lys²⁸), Gly en position 29 et 30 (Gly²⁹ et Gly³⁰) et Thr en position 31 (Thr³¹), X1 représentant Lys (Lys²¹) ou Nle (Nle²¹) et X2 représentant Asp (Asp²⁵) ou Asn (Asn²⁵).

Un certain nombre de ces analogues cycliques du VIP ont notamment été décrits dans la Demande de Brevet Européen 0 536 741 (HOFFMAN-LAROCHE). Un analogue cyclique du VIP est un peptide dans lequel la chaîne latérale d'un des acides aminés dudit peptide est attaché, de manière covalente, à la chaîne latérale d'un autre acide aminé dudit peptide. Plus de 70 analogues cycliques du VIP sont décrits dans cette Demande de Brevet européen n° 0 536 741.

Il a été montré qu'ils ont une action intéressante sur la relaxation du muscle lisse trachéo-bronchial et qu'en outre, ils présentent une biodisponibilité significativement améliorée par rapport au VIP. En outre, ils ne présentent pas d'effets indésirables cardiovasculaires.

Les Inventeurs ont trouvé, de manière surprenante, que ces différents analogues du VIP ont aussi bien un effet protecteur que curatif sur les lésions cérébrales mimant les lésions observées chez le nouveau-né à terme ou chez le prématuré, de préférence chez le prématuré et qu'ils pouvaient être administrés par voie systémique (voie parentérale ou voie orale, par exemple), car ils passent effectivement la barrière hématoencéphalique.

Selon un mode de réalisation avantageux de l'Invention, ledit analogue de VIP cyclique présente de préférence l'une des séquences suivantes :

Ac-HSDAVFTENYTKLRKQNleAAKKYLNDLKKGGT-NH2 (SEQ ID NO:2)

Ac-HSDAVFTENYTKLRKRNleAAKKYLNDLKKGGT-NH2 (SEQ ID NO:3)

Selon un autre mode de réalisation avantageux de l'invention, l'analogue du VIP non cyclique selon l'invention présente de préférence l'une des séquences suivantes :

Ac-HSDAVFTENYTKLRKQNleAAKKYLNDLKKGGT-NH2 (SEQ ID NO:4)

Ac-HSDAVFTENYTKLRKQNleAAKNleYLNNLKKGGT-NH2 (SEQ ID NO:5)

Alors que le VIP injecté par voie intracérébrale protège le cerveau de la souris contre les lésions induites par l'iboténate au niveau du cortex cérébral à J_{N} et de la substance blanche à J_{N} + 5, les différents analogues VIP protègent par voie systémique (voie intrapéritonéale dans le cas de la souris), de manière significative, la substance blanche à J_{N} + 5 et le cortex à J_{N}.

De manière surprenante, ces effets à J_{N} + 5 s'observent si les analogues du VIP sont administrés dans les 8 à 12 heures après l'injection d'iboténate, ce qui signifie qu'ils peuvent être utilisés en traitement curatif.

De manière surprenante, les Inventeurs ont trouvé que de tels dérivés protègent le cerveau en développement, surtout la substance blanche, contre des lésions cérébrales ressemblant aux lésions du nouveau-né prématuré, et dans une moindre mesure du nouveau-né à terme ou à certaines lésions ischémiques touchant le cerveau foetal.

Selon un autre mode de réalisation avantageux de l'invention, ledit analogue de VIP est destiné à être administré à une dose unitaire comprise entre 2 à 5 mg/kg, de préférence de 4 mg/kg par voie parentérale ou par voie orale.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

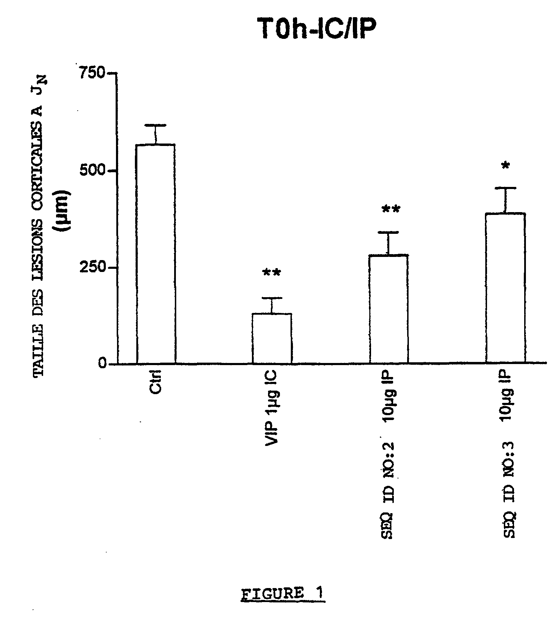

- la figure 1 illustre les résultats comparatifs obtenus avec le VIP (SEQ ID NO:1) et deux VIP cycliques respectivement de SEQ ID NO:2 et SEQ ID NO:3, sur la taille des lésions corticales en µm, lorsque l'iboténate est administré à J_{N} ;

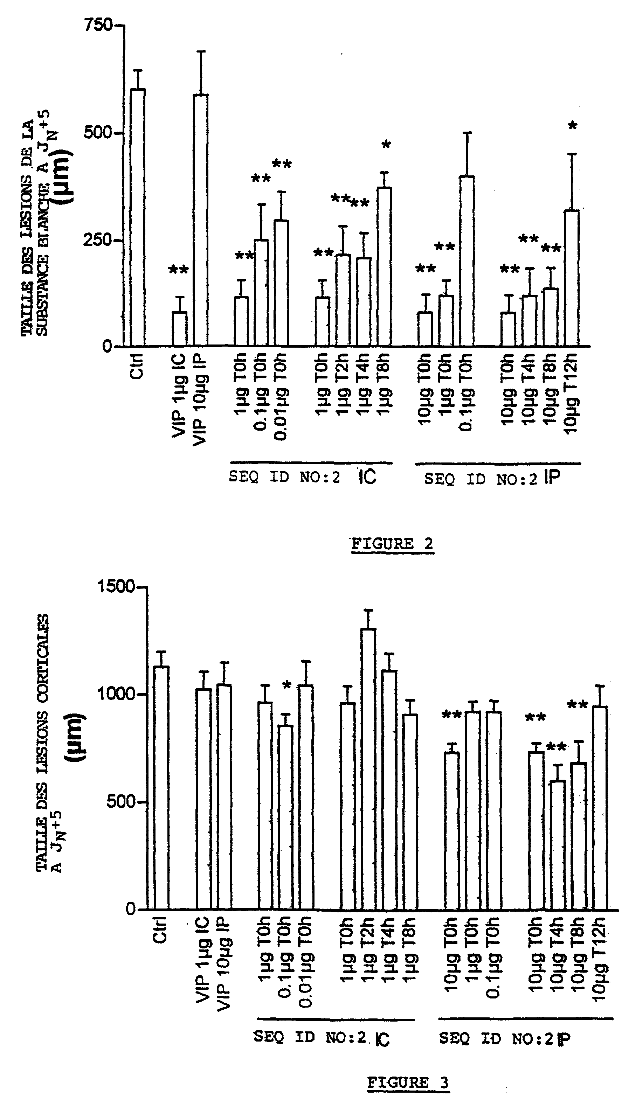

- les figures 2 et 3 illustrent l'effet du VIP cyclique de SEQ ID NO:2 sur les lésions corticales et sur les lésions de substance blanche, cinq jours après la naissance (J_{N}+5) ; les injections sont effectuées soit par voie intracérébrale, soit par voie intrapéritonéale, simultanément ou après l'injection d'iboténate ;

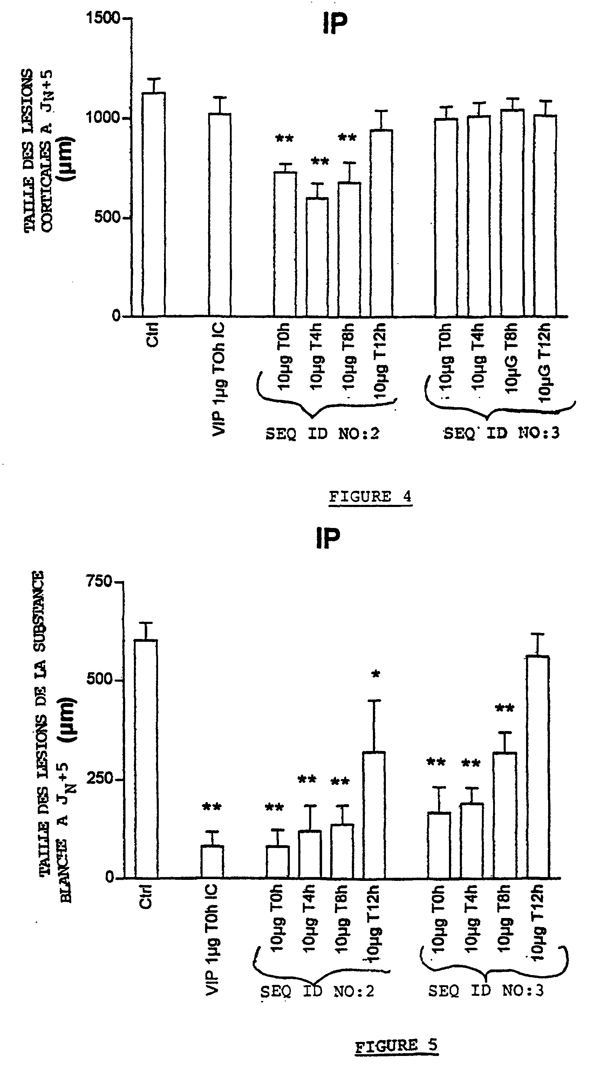

- les figures 4 et 5 correspondent à une étude comparée des effets des analogues de VIP cyclique de SEQ ID NO:2 et de SEQ ID NO:3 administrés par voie intrapéritonéale par rapport au VIP injecté par voie intracérébrale à J_{N}+5 : effets sur les lésions corticales (figure 4) et effets sur les lésions de substance blanche (figure 5).

Dans l'ensemble de ces figures : * = p<0,05, ** = p<0,01 (ANOVA + test de comparaison multiple de Dunnett par rapport aux contrôles (iboténate seul)).

Il doit être bien entendu, toutefois, que ces exemples sont donnés uniquement à titre d'illustration de l'objet de l'invention, dont ils ne constituent en aucune manière une limitation.

### EXEMPLE:

Les souris sont préparées comme décrit dans *J Clin. Investig*. précité.

Le VIP est injecté par voie intracérébrale à raison de 1 µg (dose par animal) et les VIP cycliques ont été injectés par voie intrapéritonéale à raison de 10 µg (dose par animal).

On observe :
- à J_{N}, c'est-à-dire le jour de la naissance, une diminution significative de la taille des lésions corticales, exprimées en µm, lorsque l'iboténate est administré en même temps que le VIP ou l'un des analogues cycliques du VIP ;
- à J_{N}+5, une diminution de la taille des lésions de substance blanche significative aussi bien après injection par voie intracérébrale que par voie intrapéritonéale de l'analogue cyclique du VIP de SEQ ID NO:2 (figure 2), alors que la diminution de la taille des lésions corticales, exprimée en µm (figure 3) n'est significative que lorsque l'analogue cyclique du VIP de SEQ ID NO:2 est administré par voie parentérale.

La figure 2 montre également que le VIP administré par voie intrapéritonéale ne présente aucune activité sur les lésions de substance blanche, alors qu'aux mêmes doses l'analogue cyclique du VIP de SEQ ID NO:2 présente une activité significative.

Ces résultats montrent un effet particulièrement significatif du VIP cyclique de SEQ ID NO:2 dans la neuroprotection de la substance blanche.

Ces résultats sont particulièrement intéressants du fait qu'un tel modèle reproduit les lésions du prématuré humain.

Les figures 4 et 5 montrent que l'analogue cyclique du VIP de SEQ ID NO:2, injecté par voie intracérébrale ou par voie intrapéritonéale, protège significativement la substance blanche et assure une certaine protection du cortex cérébral, surtout en intrapéritonéale, à J_{N}+5.

Ces effets à J_{N}+5 s'observent si l'analogue cyclique du VIP de SEQ ID NO:2 est administré dans les 8 à 12 premières heures après l'injection d'iboténate ; ceci signifie qu'on dispose chez l'homme d'un délai de 12 heures pour agir en cas de lésion cérébrale. Le traitement dans ce laps de temps est important.

Ces résultats montrent également que l'analogue cyclique de VIP de SEQ ID NO:3, injecté par voie intrapéritonéale, reproduit les effets neuroprotecteurs de l'analogue cyclique de VIP de SEQ ID NO:2, tant au niveau du cortex à J_{N} que de la substance blanche à J_{N}+5.

L'ensemble de ces résultats montrant que l'analogue cyclique de VIP de SEQ ID NO:2 et son dérivé, l'analogue cyclique de VIP de SEQ ID NO:3 possèdent des propriétés anti-excitotoxiques permettant, dans un modèle animal *in vivo*, de protéger le cerveau en développement (surtout la substance blanche) contre des lésions cérébrales ressemblant aux lésions du nouveau-né prématuré ou à certaines lésions ischémiques touchant le cerveau foetal.

## Revendications

1. Utilisation d'un analogue du VIP sélectionné dans le groupe constitué par les analogues cycliques du VIP et des dérivés du VIP non cycliques de 31 aminoacides comprenant en position N-terminale un groupe N-acétyle, puis Glu en position 8 (Glu⁸), Lys en position 12 (Lys¹²), norleucine en position 17 (Nle¹⁷), Ala en position 19 (Ala¹⁹), X1 en position 21, X2 en position 25, Leu en position 26 (Leu²⁶), Lys en positions 27 et 28 (Lys²⁷ et Lys²⁸), Gly en position 29 et 30 (Gly²⁹ et Gly³⁰) et Thr en position 31(Thr³¹), X1 représentant Lys (Lys²¹) ou Nle (Nle²¹) et X2 représentant Asp (Asp²⁵) ou Asn (Asn²⁵), pour l'obtention d'un médicament pour la prévention ou le traitement des lésions cérébrales du foetus, du nouveau-né prématuré ou à terme ou du jeune nourrisson humains.

2. Utilisation selon la revendication 1, **caractérisée en ce que** ledit analogue de VIP est destiné à être administré par voie systémique.

3. Utilisation selon l'une quelconque des revendications 1 ou 2, **caractérisée en ce que** ledit analogue de VIP cyclique présente de préférence l'une des séquences suivantes :

Ac-HSDAVFTENYTKLRKQNleAAKKYLNDLKKGGT-NH2 (SEQ ID NO:2)

Ac-HSDAVFTENYTKLRKRNleAAKKYLNDLKKGGT-NH2 (SEQ ID NO:3)

4. Utilisation selon l'une quelconque des revendications 1 ou 2, **caractérisée en ce que** l'analogue du VIP non cyclique présente de préférence l'une des séquences suivantes:

Ac-HSDAVFTENYTKLRKQNleAAKKYLNDLKKGGT-NH2 (SEQ ID NO:4)

Ac-HSDAVFTENYTKLRKQNleAAKNleYLNNLKKGGT-NH2 (SEQ ID NO:5)

5. Utilisation selon l'une quelconque des revendications précédentes, **caractérisée en ce que** dans le traitement des lésions cérébrales de la substance blanche du nouveau-né humain prématuré, ledit médicament est destiné à être administré dans les 8 à 12 heures après l'apparition desdites lésions.

6. Utilisation selon l'une quelconque des revendications précédentes, **caractérisée en ce que** ledit médicament est destiné à être administré à une dose unitaire comprise entre 2 à 5 mg/kg, de préférence de 4 mg/kg, par voie parentérale ou par voie orale.

## Claims

1. Use of a VIP analogue, selected from the group consisting of cyclic VIP analogues and non-cyclic VIP derivatives of 31 amino acids comprising an N-acetyl group in N-terrninal position, then Glu in position 8 (Glu⁸), Lys in position 12 (Lys¹²), norleucine in position 17 (Nle¹⁷), Ala in position 19 (Ala¹⁹), X1 in position 21, X2 in position 25, Leu in position 26 (Leu²⁶), Lys in positions 27 and 28 (Lys²⁷ and Lys²⁸), Gly in positions 29 and 30 (Gly²⁹ and Gly³⁰) and Thr in position 31 (Thr³¹), X1 representing Lys (Lys²¹) or Nle (Nle²¹) and X2 representing Asp (Asp²⁵) or Asn (Asn²⁵), to obtain a drug for the prevention or treatment of cerebral lesions in the human foetus, premature or full-term neonate or young infant.

2. Use in accordance with Claim 1, **characterized in that** the VIP analogue is intended for systemic administration.

3. Use in accordance with either of Claims 1 or 2, **characterized in that** the cyclic VIP analogue ideally presents one of the following sequences:

Ac-HSDAVFTENYTKLRKQN1eAAKKYLNDLKKGGT-NH2 (SEQ ID NO:2)

Ac-HSDAVFTENYTKLRKRN1eAAKKYLNDLKKGGT-NH2 (SEQ ID NO:3)

4. Use in accordance with either of Claims 1 or 2, **characterized in that** the non-cyclic VIP analogue ideally presents one of the following sequences:

Ac-HSDAVFTENYTKLRKQN1eAAKKYLNDLKKGGT-NH2 (SEQ ID NO:4)

Ac-HSDAVFTENYTKLRKQN1eAAKN1eYLNNLKKGGT-NH2 (SEQ ID NO:5)

5. Use in accordance with any of the previous Claims, **characterized in that**, in the treatment of lesions of the cerebral white matter in the premature human neonate, the drug is intended for administration within 8 to 12 hours of the appearance of such lesions.

6. Use in accordance with any of the previous Claims, **characterized in that** the drug is intended for administration as a metered dose of between 2 and 5 mg/kg, ideally of 4 mg/kg, either parenterally or orally.

## Patentansprüche

1. Verwendung eines VIP-Analogs, ausgewählt aus der Gruppe bestehend aus cyclischen VIP-Analogen und nicht-cyclischen VIP-Derivaten von 31 Aminosäuren, die in der N-terminalen Position eine N-Acetylgruppe aufweisen, des weiteren Glu in Position 8 (Glu⁸) Lys in Position 12 (Lys¹²), Norleucin in Position 17 (Nle¹⁷), Ala in Position 19 (Ala¹⁹), X1 in Position 21, X2 in Position 25, Leu in Position 26 (Leu²⁶), Lys in Positionen 27 und 28 (Lys²⁷ und Lys²⁸), Gly in Position 29 und 30 (Gly²⁹ und Gly³⁰) und Thr in Position 31 (Thr³¹), wobei X1 für Lys (Lys²¹) oder Nle (Nle²¹) steht, und X2 für Asp (Asp²⁵) oder Asn (Asn²⁵) steht, für die Herstellung eines Medikaments zur Verhinderung bzw. Behandlung zerebraler Läsionen des menschlichen Fötus, vorzeitig oder rechtzeitig geborenen Neugeborenen, oder Kleinsäuglings.

2. Verwendung nach Anspruch 1, **dadurch gekennzeichnet, daß** das VIP-Analog zur Verabreichung auf systemischem Wege vorgesehen ist.

3. Verwendung nach einem der Ansprüche 1 oder 2, **dadurch gekennzeichnet, daß** das cyclische VIP-Analog vorzugsweise eine der beiden folgenden Sequenzen aufweist:

Ac-HSDAVFTENYTKLRKQNleAAKKYLNDLKKGGT-NH2 (SEQ-ID Nr. 2)

Ac-HSDAVFTENYTKLRKRNleAAKKYLNDLKKGGT-NH2 (SEQ-ID Nr. 3)

4. Verwendung nach einem der Ansprüche 1 oder 2, **dadurch gekennzeichnet, daß** das nicht-cyclische VIP-Analog vorzugsweise eine der beiden folgenden Sequenzen aufweist:

Ac-HSDAVFTENYTKLRKQNleAAKKYLNDLKKGGT-NH2 (SEQ-ID Nr. 4)

Ac-HSDAVFTENYTKLRKQNleAAKNleYLNNLKKGGT-NH2    (SEQ-ID Nr. 5)

5. Verwendung nach einem der vorhergehenden Ansprüche, **dadurch gekennzeichnet, daß** bei der Behandlung von zerebralen Läsionen der weißen Substanz des frühgeborenen menschlichen Neugeborenen das Medikament dazu vorgesehen ist, in den 8 bis 12 Stunden nach dem Auftreten der Läsionen verabreicht zu werden.

6. Verwendung nach einem der vorhergehenden Ansprüche, **dadurch gekennzeichnet, daß** das Medikament dazu vorgesehen ist, in einer Einheitsdosis von zwischen 2 bis 5 mg/kg, vorzugsweise von 4 mg/kg, auf parenteralem oder oralem Wege verabreicht zu werden
